(19) Europäisches Patentamt
European Patent Office
Office européen des brevets

(11) Veröffentlichungsnummer: **0 086 416**
**A2**

(12) **EUROPÄISCHE PATENTANMELDUNG**

(21) Anmeldenummer: **83101098.8**

(22) Anmeldetag: **05.02.83**

(51) Int. Cl.³: **C 07 D 471/14**, A 61 K 31/435
//
(C07D471/14, 221/00,
221/00, 209/00)

(30) Priorität: **12.02.82 DE 3204960**

(43) Veröffentlichungstag der Anmeldung: **24.08.83**
**Patentblatt 83/34**

(84) Benannte Vertragsstaaten: **AT BE CH DE FR GB IT LI LU NL SE**

(71) Anmelder: **Dr. Willmar Schwabe GmbH & Co.,**
**Willmar-Schwabe-Strasse 4, D-7500 Karlsruhe 41 (DE)**

(72) Erfinder: **Jaggy, Hermann Ernst Walter Dr. Dipl.-Chem.,**
**Kapellenweg 7, D-7525 Bad Schönborn 1 (DE)**
Erfinder: **Sunder Chatterjee, Shyam Dr. Dipl.-Chem.,**
**Werrabronner Strasse 8a, D-7500 Karlsruhe 41 (DE)**
Erfinder: **Gabard, Bernard Louis, Dr. Dipl.-Ing.,**
**Gürrichstrasse 44, D-7500 Karlsruhe 31 (DE)**

(74) Vertreter: **Bunke, Holger, Dr. Dipl.-Chem. et al,**
**Patentanwälte Prinz, Bunke & Partner Lessingstrasse 9,**
**D-7000 Stuttgart 1 (DE)**

(54) **Corynanthein-Derivate, Verfahren zu ihrer Herstellung und ihre Verwendung als Arzneimittel.**

(57) Corynanthein-Derivate der allgemeinen Formel I, worin R ein Wasserstoff- oder Alkaliatom, $NH_4$– oder den Rest eines Amins oder eine Alkylgruppe mit 1 bis 4 C-Atomen bedeutet, sowie deren physiologisch unbedenkliche Säureadditionssalze.

Verfahren zur Herstellung dieser Verbindungen und deren Verwendung als pharmazeutische Zubereitungen sowie die Verwendung von Dihydrocorynanthein als Antihypertonikum.

EP 0 086 416 A2

0086416

P 2596 EP 4.2.1983

Firma Dr. Willmar Schwabe GmbH & Co.
Willmar-Schwabe-Straße 4
7500 Karlsruhe 41

# Corynanthein-Derivate, Verfahren zu ihrer Herstellung und ihre Verwendung als Arzneimittel

Die Erfindung betrifft Corynanthein-Derivate der allgemeinen Formel I, insbesondere Tetrahydrocorynanthein (THCN), einschließlich der pharmakologisch unbedenklichen Säureadditionssalze dieser Verbindungen, sowie Verfahren zu deren Herstellung und diese Verbindungen enthaltende pharmazeutische Zubereitungen, d. h. die Verwendung der Corynanthein-Derivate als Arzneimittel.

Zur Behandlung von Durchblutungsstörungen und für die Therapie des Bluthochdruckes stehen eine Vielzahl von im Handel befindlichen Arzneimittelspezialitäten zur Verfügung. Trotzdem gibt es immer noch eine Vielzahl

von Behandlungsfällen, bei denen die zur Verfügung stehenden Präparate versagen oder aufgrund ihrer spezifischen Nebenwirkungen oder ihres zeitlichen Wirkungsablaufs unbefriedigend sind.

So wird beispielsweise in der Therapie von Durchblutungsstörungen seit Jahrzehnten Raubasin (=Ajmalicin = δ-Yohimbin) als Wirksubstanz verwendet, die den Nachteil einer relativ kurzen Wirkungsdauer besitzt und deshalb eine ständige Wiederholung der Applikation in kurzen Zeitabständen erforderlich macht.

Es besteht deshalb immer noch ein Bedürfnis nach pharmakologisch wirksamen Verbindungen und Arzneimitteln, die eine lang anhaltende blutdrucksenkende Wirksamkeit besitzen, gleichzeitig aber möglichst weitgehend frei von unangenehmen Nebenwirkungen sind.

Der Erfindung liegt die Aufgabe zugrunde, das Bedürfnis nach der Bereitstellung neuer pharmakologisch wirksamer Verbindungen und Arzneimittel, insbesondere neuer Antihypertonika, zu befriedigen, und zwar vorzugsweise solcher, die bei äquivalenter Dosierung eine stärkere und länger anhaltende Blutdrucksenkung bewirken und ein günstigeres pharmakologisches Profil aufweisen als Raubasin.

Diese Aufgabe wird durch die Bereitstellung der erfindungsgemäßen Stoffe, durch Verfahren zur Herstellung dieser Stoffe und durch deren Verwendung als Arzneimittel oder als Bestandteil pharmazeutischer Zubereitungen gelöst.

Gegenstand der Erfindung sind somit die Corynanthein-Derivate der allgemeinen Formel I,

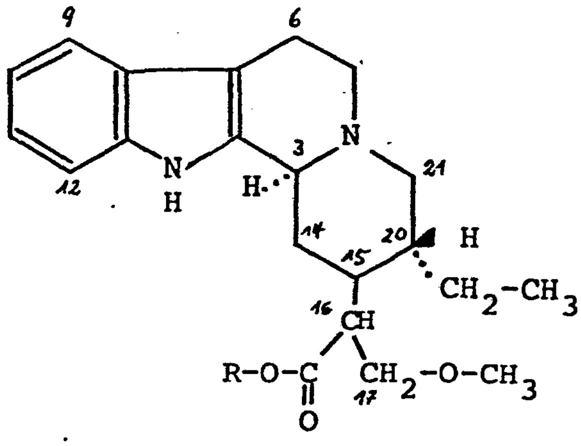

(I)

worin R ein Wasserstoff- oder Alkaliatom, $NH_4$- oder den Rest eines Amins oder eine Alkylgruppe mit 1 bis 4 C-Atomen bedeutet, sowie deren physiologisch unbedenkliche Säureadditionssalze.

Die erfindungsgemäßen Verbindungen besitzen neben einer ausgezeichneten blutdrucksenkenden Wirkung eine ausgeprägte vasodilatatorische und das Zentralnervensystem beeinflussende Wirksamkeit. Die Wirkungsdauer der erfindungsgemäßen Verbindungen ist signifikant länger als diejenige des bekannten Raubasins. Dagegen konnten unangenehme Nebenwirkungen bei den erfindungsgemäßen Verbindungen bisher nicht festgestellt werden.

Die erfindungsgemäßen Verbindungen können durch Partialsynthese, nämlich durch katalytische Hydrierung der Naturstoffe Corynanthein (Formel IIa) und Dihydrocorynanthein (Formel IIb), durch katalytische Hydrierung von Gemischen aus Corynanthein und Dihydrocorynanthein oder von diese schwach basischen Indolalkaloide enthaltendem natürlichem pflanzlichem Material hergestellt werden.

Die natürlich, nämlich in der Rinde von Pseudocinchona africana A. Cheval (syn. Corynanthe pachyceras) und Corynanthe yohimbe K. Schum., vorkommenden Indolalkaloide Corynanthein (IIa) und Dihydrocorynanthein (IIb) der allgemeinen Formel II

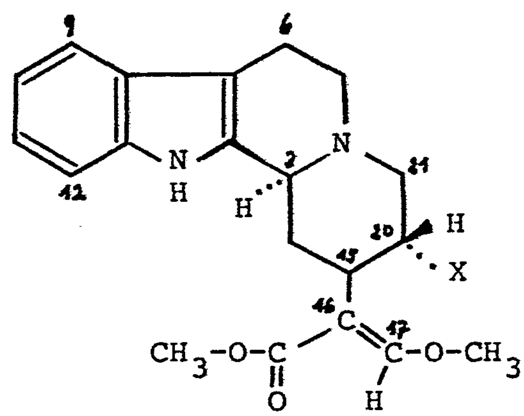

(II)

IIa: X = $CH{=}CH_2$

IIb: X = $CH_2-CH_3$

sind bekannte Naturstoffe und als solche beschrieben in Helv. Chim. Acta. 9, 1059 (1926) und Helv. Chim. Acta 35, 851 (1952). Die Konstitution des Corynantheins ist bekannt aus Helv. Chim. Acta 34, 1207 (1951), die absolute Konfiguration des Dihydrocorynantheins, und damit auch diejenige des Corynantheins, ist bekannt aus Tetrahedron Letters 39, 9457 (1965). Bekannt ist weiterhin die katalytische Hydrierung von Corynanthein und damit dessen Überführung in Dihydrocorynanthein, und zwar mittels Palladium auf Bariumsulfat (Helv. Chim. Acta 35, 851 (1952)) und mittels Palladium auf Bariumcarbonat (Compt. rend. hebd. Seance Acad. Sci. 234, 1562 (1952)).

Über eine etwaige pharmakologische Wirksamkeit oder eine therapeutische Verwendungsmöglichkeit der Naturstoffe Corynanthein und Dihydrycorynanthein ist dagegen bisher nichts bekannt geworden. Beschrieben ist lediglich, daß Zubereitungen aus der Rinde von Pseudocinchona in der afrikanischen Volksmedizin gegen Husten, Lepra und Fieber benutzt werden (F.R.Irvine, "Woody Plants of Ghana", Oxford University Press 1961, S. 665).

Gleichgültig, ob bei der Herstellung der erfindungsgemäßen Verbindungen von Reinsubstanzen Corynanthein oder Dihydrocorynanthein, von deren Gemischen oder von diese beiden Indolalkaloide sowie ggfs. Corynantheidin enthaltendem natürlichem Material, das beispielsweise aus Pseudocinchona africana gewonnen wurde, ausgegangen wird, verläuft die katalytische Hydrierung so, daß zunächst die exo-ständige Vinylgruppe des Corynantheins zur entsprechenden exo-ständigen Äthylgruppe aufhydriert wird, wodurch (exo-) Dihydrocorynanthein entsteht.

Das als Zwischenprodukt gebildete Dihydrocorynanthein, das erfindungsgemäß nicht isoliert zu werden braucht,

wird bei Fortsetzung der katalytischen Hydrierung an der zwischen C-16 und C-17 bestehenden Doppelbindung hydriert, so daß die erfindungsgemäße Verbindung der Formel I, worin R eine Methylgruppe ist, entsteht, die hier "Tetrahydrocorynanthein" (THCN) genannt werden soll.

Die katalytische Hydrierung wird erfindungsgemäß stets bis zur Sättigung durchgeführt, also solange, bis kein weiterer Wasserstoff mehr aufgenommen wird. Wenn das zu hydrierende Ausgangsmaterial Corynanthein enthält, sind zur Herstellung des erfindungsgemäßen Tetrahydrocorynantheins zwei äquivalente Wasserstoff erforderlich, wenn das Ausgangsmaterial jedoch nur Dihydrocorynanthein enthält, so ist zur Herstellung von THCN nur ein Äquivalent Wasserstoff nötig.

Die Hydrierung wird vorzugsweise in alkoholischer oder wäßrig-alkoholischer Lösung der Ausgangssubstanzen Corynanthein und/oder Dihydrocorynanthein durchgeführt, wobei vor allem die niederen Alkohole als Lösemittel verwendet werden.

Als Katalysatoren verwendet man erfindungsgemäß Edelmetallkatalysatoren (Pd, Pt, Rh), aber auch andere Metallkatalysatoren, jeweils in fein verteilter Form, auf üblichen inerten organischen oder anorganischen Trägermaterialien, vorzugsweise Pd/Kohle- oder Ni-Katalysatoren (z. B. Raney-Nickel oder Ni auf Kieselgur).

Die Hydrierung wird vorzugsweise bei einem Druck von 50 bis 110 bar und bei Temperaturen zwischen 20 und 100°C durchgeführt.

Das nach dem erfinderungsgemäßen Verfahren aus Corynanthein und/oder Dihydrocorynanthein entstandene Tetrahydrocorynanthein ist kein Gemisch der an C-16

möglichen Enantiomeren, sondern eine einheitliche Verbindung. Dies ergibt sich zum einen aus dem $^{13}C$-NMR-Spektrum (vergl. Beispiel 1), und zum anderen daraus, daß das unter verschiedenen Reaktionsbedingungen, auch bei Variation von Lösemittel und Katalysator, erhaltene Tetrahydrocorynanthein stets denselben optischen Drehwert hat. Ein weiteres Indiz für das Vorliegen nur eines der theoretisch möglichen Isomeren ist die Tatsache, daß bei der Dünnschichtchromatographie in verschiedenen Laufmittel-Systemen jeweils nur ein Fleck erhalten wurde. Ein Enantiomerengemisch, das ja ein Diastereomerengemisch ist, müßte sich dünnschichtchromatographisch auftrennen lassen, so, wie dies bei den diastereomeren Verbindungen Corynantheidin und Dihydrocorynanthein ist.

Das nach dem vorstehend beschriebenen Verfahren hergestellte Tetrahydrocorynanthein der allgemeinen Formel I, worin R eine Methylgruppe ist, kann wie folgt in die übrigen Verbindungen der allgemeinen Formel I umgewandelt werden: Es kann nach an sich bekannten Methoden zur freien Säure (R = H) verseift und ggfs. mit anorganischen Basen oder physiologisch verträglichen Aminen in ihre Salze übergeführt werden oder es kann nach ebenfalls bekannten Methoden umgeestert werden, also in Verbindungen der allgemeinen Formel I umgewandelt werden, worin R eine Äthyl-, Propyl-, Isopropyl-, Butyl-, Isobutyl- oder tert.-Butyl-Gruppe bedeutet. Die zuletzt genannten Alkylester können aber auch auf dem Weg über die freie Säure durch Umsetzung dieser Säure mit einem entsprechenden Alkanol mit 2 bis 4 C-Atomen hergestellt werden.

Die erfindungsgemäßen Verbindungen werden dadurch in ihre physiologisch unbedenklichen Salze übergeführt, daß man ihre Lösungen in geeigneten Lösemitteln oder gleich die nach dem Abtrennen des Katalysators verbleibenden Reaktionslösungen mit anorganischen Säuren (Halogenwasserstoffsäure, Schwefelsäure, Salpetersäure, Phosphorsäure und dergl.) oder mit starken organischen Säuren (z.B. Oxalsäure oder Methansulfonsäure) versetzt. Das erfindungsgemäße Tetrahydrocorynanthein ist gegenüber Säuren und anderen Agenzien erstaunlich stabil, vor allem wesentlich stabiler als Dihydrocorynanthein mit seiner Enol-Äther-Gruppe.

Gegenstand der Erfindung ist ferner eine pharmazeutische Zubereitung, die neben üblichen Träger- und Zusatzstoffen eine oder mehrere der erfindungsgemäßen Verbindungen oder ihrer physiologisch unbedenklichen Salze enthält. Übliche Trägerstoffe sind beispielsweise Wasser, pflanzliche Öle, Polyäthylenglykole, Glycerinester, Gelatine, Kohlenhydrate wie Laktose oder Stärke, Magnesiumstearat, Talk. Übliche Zusatzstoffe sind beispielsweise Konservierungs-, Stabilisierungs-, Gleit-, Netzmittel, Emulgatoren, physiologisch unbedenkliche Salze, Puffersubstanzen, Farb-, Geschmacks- und Aromastoffe. Die Auswahl der Träger- und Zusatzstoffe hängt davon ab, ob die erfindungsgemäßen Verbindungen enteral oder parenteral appliziert werden sollen.

Die erfindungsgemäße pharmazeutische Zubereitung kann als Arzneimittel in der Human- und Veterinärmedizin verwendet werden, insbesondere als Antihypertonikum. Die erfindungsgemäßen Verbindungen können auch in Kombination mit anderen, den Blutdruck senkenden bekannten Wirkstoffen verabreicht werden, beispielsweise mit Saluretika, Hydrazinophthalazinen und Beta-Blockern. Die Dosierung von Dihydrocorynanthein und von Tetrahydrocorynanthein oder eines ihrer Säureadditionssalze liegt

bei oraler Applikation bei 5 bis 20 mg, 2 bis 3 mal täglich, bei intravenöser Applikation dagegen 2 bis 3 mal 1 bis 5 mg der entsprechenden freien Base oder einer äquivalenten Menge eines ihrer Säureadditionssalze.

Gegenstand der Erfindung ist schließlich die Verwendung von Dihydrocorynanthein (DHCN) als Antihypertonikum.

Zur Herstellung von Tabletten mit jeweils 100 mg Einzelgewicht, die 10 mg THCN-Base oder eine äquivalente Menge eines Säureadditionssalzes enthalten, benötigt man

1. 10 g Tetrahydrocorynanthein bzw. eine äquivalente Menge eines seiner Säureadditionssalze
2. 49 g mikrokristalline Cellulose
3. 20 g Milchzucker
4. 20 g Maisstärke
5. 0,5 g kolloidale Kieselsäure
6. 0,5 g Magnesiumstearat.

Die Substanzen 1. bis 4. werden trocken 10 Min. lang gemischt, anschließend wird das Gemisch der Substanzen 5. und 6. zugegeben, man mischt weitere 10 Minuten und verpreßt das so erhaltene Pulver auf einer Tablettiermaschine zu Tabletten von 100 mg Einzelgewicht.

Die in den folgenden Beispielen enthaltenen Abkürzungen haben folgende Bedeutung:

Schmp. = Schmelzpunkt (unkorrigiert)
(Beispiele 1 bis 8 und 17 aufgenommen im Schmelzpunktsröhrchen, Beispiele 9 bis 16 aufgenommen auf dem Schmelzpunktsmikroskop)

Zers. = Zersetzung

Subl. = Sublimation

$[\alpha]_D^{20}$ = optische Drehung bei 20°C, Natrium-D-Linie.

Hinter den optischen Drehwerten sind die Konzentrationen der gemessenen Lösungen angegeben, wobei c 2 beispielsweise eine Konzentration von 2 g/100 ml Lösung bedeutet; das Lösungsmittel ist jeweils gesondert angegeben.

Alle Temperaturen sind in °C angegeben.

Die folgenden Beispiele 1 bis 8 betreffen die Herstellung des erfindungsgemäßen "Tetrahydrocorynantheins" (= THCN), also der Verbindung der allgemeinen Formel I, worin R eine Methylgruppe ist, und zwar unter Verwendung verschiedener Ausgangsmaterialien und verschiedener Katalysatoren. Die Beispiele 9 bis 16 betreffen die Herstellung verschiedener Säureadditionssalze des THCN. Beispiel 17 betrifft die Herstellung der erfindungsgemäßen Verbindung der allgemeinen Formel I, worin R ein Wasserstoffatom ist.

Beispiel Nr. 1

Hydrierung eines Alkaloid-Gemisches:

30 g des Chloroform-Extraktes aus der Rinde von Pseudocinchona africana werden in 500 ml Essigester gelöst und zweimal mit jeweils 250 ml 1 N-Salzsäure ausgeschüttelt. Nach dem Alkalisieren der salzsauren Lösung mit 25 % Ammoniaklösung auf pH = 8 wird dreimal mit jeweils 250 ml Essigester ausgeschüttelt. Die vereinigten Essigester-Phasen werden mit 300 ml Wasser gewaschen, über Natriumsulfat getrocknet und im Vakuum zur Trockne eingeengt. Der Rückstand wiegt 23 g und enthält 76,6 % Alkaloide des Corynanthein-Typs, bestehend aus 28,6 % Corynantheidin, 35,8 % Corynanthein und 12,2 % Dihydrocorynanthein.

20 g Alkaloid-Gemisch werden in 100 ml Isopropanol / Wasser 80 + 20 Volumenteile gelöst. Nach Zugabe von 4 g 10 % - Palladium auf Aktivkohle in 200 ml Isopropanol / Wasser 80 + 20 wird in einem Hochdruckautoklaven mit einem Wasserstoffdruck von 70 bar unter Schütteln und externer Heizung auf 80 - 90°C 80 Stunden hydriert. (Die Umsetzung von Corynanthein und Dihydrocorynanthein zu Tetrahydrocorynanthein kann durch Dünnschichtchromatographie auf Kieselgel geprüft werden: im Fließmittel Toluol / Methylenchlorid / Isopropanol 75 + 20 + 5 Volumenteile + 5 Tropfen 25 %-Ammoniaklösung ergeben sich folgende Rf-Werte: Corynanthein Rf = 0,41, Dihydrocorynanthein Rf = 0,39, Tetrahydrocorynanthein Rf = 0,37 und Corynantheidin Rf = 0,60. Nach Anfärbung mit Vanillin / $H_3PO_4$ und Erwärmen der DC-Platte ca. 5 Minuten auf 120°C zeigen die Substanzflecken folgende Farben:

Corynanthein : kräftig Blauviolett,
Dihydrocorynanthein : Grauviolett,
Tetrahydrocorynanthein : kräftig Blauviolett,
Corynantheidin : Blaßviolett Rosa.

Nach beendeter Hydrierung wird der Katalysator abfiltriert. Das Filtrat ergibt nach dem Eindampfen im Vakuum 19,8 g Rückstand. Dieser wird in 80 ml 95 %-Äthanol in der Siedehitze gelöst, die Lösung wird heiß filtriert und nach dem Abkühlen auf Raumtemperatur in den Kühlschrank gestellt. Die ausgefallenen Kristalle werden abgesaugt. Aus der konzentrierten Mutterlauge kristallisiert weitere Substanz. Insgesamt werden 3,5 g kristallines THCN erhalten.

Die Mutterlauge aus der zweiten Kristallisation (15,2 g) kann über eine Säule aus 750 g Kieselgel, das mit 5% seines Gewichtes einer 25 %-Amoniaklösung alkalisiert wurde, im Lösungsmittelsystem Toluol/Essigester - 80/20 fraktioniert werden. Es werden 2,3 g amorphes Roh-THCN erhalten, das beim Umkristallisieren aus 95 %-Äthanol 1,2 g kristallines THCN ergibt.

Die Ausbeute beträgt 48,5 %, bezogen auf die Summe des eingesetzten Corynantheins und des eingesetzten Dihydrocorynantheins.

Schmp.: 215 bis 226°C (zum Teil sublimiert die Substanz unzersetzt ab 175°C).

$[\alpha]^{20}_{D}$ : - 25° (c2; Chloroform)

IR-Spektrum (KBr): 1720 (CO), 3365 $cm^{-1}$ (NH).

Das in $CDCl_3$-Lösung bei 22,63 MHz aufgenommene $^{13}C$-Kernresonanz-Spektrum zeigt für die einzelnen C-Atome folgende chemischen Verschiebungen (in ppm, bezogen auf Tetramethylsilan als innerem Standard):

| | |
|---|---|
| C - 2 | 134,908 |
| C - 3 | 60,145 bzw. 59,174 |
| C - 5 | 53,348 |
| C - 6 | 21,954 |
| C - 7 | 108,422 |
| C - 8 | 127,680 |

BAD ORIGINAL

| | | |
|---|---|---|
| C - 9 | 118,294 | |
| C - 10 | 121,530 | |
| C - 11 | 119,588 | |
| C - 12 | 111,012 | |
| C - 13 | 136,364 | |
| C - 14 | 31,771 | |
| C - 15 | 40,618 | bzw. 39,916 |
| C - 16 | 45,742 | |
| C - 17 | 72,335 | |
| C - 18 | 10,896 | |
| C - 19 | 23,356 | |
| C - 20 | 40,618 | bzw. 39,916 |
| C - 21 | 60,145 | bzw. 59,174 |
| $OCH_3$ | 60,684 | |
| $COO\overset{\times}{C}H_3$ | 51,730 | |
| $\overset{\times}{C}OOCH_3$ | 173,261 | |

Für die Analyse werden 0,5 g des umkristallisierten THCN nocheinmal aus 10 ml 95 %-Äthanol umkristalliert. Man erhält reine Kristalle vom Schmp. 218 bis 219°C.
Elementaranalyse: $C_{22}H_{30}N_2O_3$ (370,50)
Ber.: C 71,32 H 8,16 N 7,56 O 12,96
Gef.: C 71,37 H 8,39 N 7,34 O 12,90

Beispiel Nr. 2:

Hydrierung eines Corynanthein/Dihydrocorynanthein-Gemisches mit Pd/Kohle:

10 g kristallines Corynanthein/Dihydrocorynanthein-Gemisch (ca. 27 mmol) werden in 100 ml n-Propanol/Wasser - 80/20 (Volumteile) gelöst. Nach Zugabe von 1,5 g 10% Pd auf Kohle in 200 ml n-Propanol/Wasser - 80/20 wird bei 60 bar Wasserstoff unter Schütteln bei 90°C Außentemperatur 82 Stunden hydriert (die Dünnschicht-Probe zeigt nur wenig nicht-hydriertes Dihydrocorynanthein). Nach dem Abfiltrieren des Katalysators wird zur Trockne

eingeengt, wobei 9,74 g Rückstand verbleiben. Aus 50 ml ml 95 %-Äthanol kristallisieren 3,12 g Tetrahydrocorynanthein, aus der Mutterlauge nochmals 0,30 g und

0,28 g. Die Auftrennung der letzten Mutterlauge über Kieselgel liefert weitere 1,2 g kristallines THCN.
Gesamtausbeute: 4,9 g Tetrahydrocorynanthein,
entspr. 48,7 % d.Theorie
Nach Umkristallisation aus 40 ml 95 % Aethanol werden erhalten 3,9 g weiße nadelförmigeKristalle vom Schmelzpunkt 216 - 218° C; $[\alpha]^{D}_{20}$ = -24,5° (c = 2 in $CHCl_3$)

Elementaranalyse: ber. für $C_{22}H_{30}N_2O_3$: MG = 370.50
C 71,32 %, H 8,16 %, N 7,56 %, O 12,96 %
gef.: C 71,79 %, H 8,46 %, N 7,42 %, O 12,33 %

Beispiel Nr. 3:

Hydrierung von Dihydrocorynanthein mit Pd/Kohle:

10 g reines kristallines Dihydrocorynanthein (27 mmol) werden in 80 ml 95 % Aethanol gelöst. Nach Zugabe von 2 g 10 % Pd auf Kohle in 10 ml 95 % Aethanol und Nachspülen mit 10 ml 95 % Aethanol wird der Autoklav verschlossen und mit Stickstoff gespült. Unter 50 bar $H_2$ wird bei 50 - 60° C 80 Stunden hydriert. Nach dem Abkühlen sind Kristalle aus dem Hydriergemisch ausgefallen. Durch Zugabe von 100 ml Methanol lösen sich die Kristalle auf, der Katalysator wird von der Lösung abfiltriert. Nach dem Eineingen zur Trockne wird aus 40 ml 95 % Aethanol kristallisiert: 7,4 g Kristalle von THCN.
Ausbeute: 19,97 mmol, entspr. 73,97 % d.Theorie
Zur Analyse wird ein Teil der Substanz nochmals aus 95 % Aethanol umkristallisiert: Schmelzpunkt: 224 - 226° C; $[\alpha]^{D}_{20}$ = -25,5° (c = 2 in $CHCl_3$)

Elementaranalyse: ber. für $C_{22}H_{30}N_2O_3$; MG = 370,0
C 71,32 %, H 8,16 %, N 7,56 %, O 12,96 %
gef.: C 71,76 %, H 8.45 %, N 7,47 %, O 12,32 %

Beispiel Nr. 4 bis 7:

Hydrierung von Dihydrocorynanthein mit verschiedenen Katalysatoren:

Jeweils 3 g Dihydrocorynanthein werden in 100 ml Isopropanol/Wasser - 80/20 oder n-Propanol/Wasser - 90/10 gelöst und 86 Stunden (Beispiel 6: 2 x 86 Std.) bei 90°C unter 60 bis 65 bar $H_2$ hydriert, wonach jeweils etwa die Hälfte des eingesetzten Dihydrocorynantheins zu THCN umgesetzt worden ist. Nach dem Abfiltrieren des Katalysators wird zur Trockne eingeengt und der Rückstand aus 10 ml 95 %-Äthanol umkristallisiert. Die verwendeten Katalysatoren, die Schmelzpunkte und die optischen Drehwerte des entstanden THCN sind in der folgenden Tabelle angegeben:

| Beisp. Nr. | 4 | 5 | 6 | 7 |
|---|---|---|---|---|
| Katalysator | $Pd/BaSO_4$ | $Pd/CaCO_3$ | Pt/Kohle | Rh/Kohle |
| Schmp. THCN | 222-226 | 222-225 | 218 | - |
| $[\alpha]_D^{20}$ | -27,75° c 0,4 $CHCl_3$ | -27,25° c 0,4 $CHCl_3$ | -25,5° c 2 $CHCl_3$ | - |

Beispiel Nr. 8:

Hydrierung eines Alkaloid-Gemisches mit Nickelkatalysator:

140 g Toluol-Extrakt aus der Rinde von Pseudocinchona africana, der wie in Beispiel 1 durch Verteilung zwischen Salzsäure und Essigester nachgereinigt wurde, wird in 1000 ml Isopropanol gelöst. Nach Zugabe von 30 g Nickelkatalysator (55 Gew.% Ni auf Kieselgur) in 200 ml Wasser und Nachspülen mit 200 ml Isopropanol wird in einem Hochdruckautoklaven mit Hubrührer bei 100 bar Wasserstoffdruck und einer Temperatur von ca. 90°C 120 Stunden hydriert. Danach wird der Katalysator abfiltriert und das Filtrat am Rotationsverdampfer unter Wasserstrahlpumpenvakuum zur Trockne eingeengt. Nach Lösen des Rückstandes in 500 ml siedendem 95 % Aethanol und Klarfiltration kristallisieren nach Abkühlung und Aufbewahrung im Kühlschrank 26 g fast reines THCN aus. Die Substanz wird nach nochmaliger Kristallisation aus 95 % Aethanol chromatographisch rein. Aus der Mutterlauge wird nach Abtrennung der Begleitstoffe über eine Kieselgel-Säule gemäß Beispiel 1 und Kristallisation aus 95 % Aethanol noch weiteres THCN erhalten.

## Beispiel Nr. 9:

Herstellung von Tetrahydrocorynanthein-Hydrochlorid:

13,3 g reines kristallines Tetrahydrocorynanthein werden in 100 ml Chloroform p.A. gelöst. Unter Kühlung im Eisbad wird HCl - Gas eingeleitet bis zur Sättigung (erkennbar am Entweichen von HCl-Dämpfen). Am Rotationsverdampfer wird unter Vakuum bei 40°C Badtemperatur zur Trockne eingeengt: 18,31 g Rückstand. Diese Substanz wird in 60 ml Methanol p.A. aufgenommen und die Lösung klar filtriert. Unter Rühren werden 180 ml eines Gemisches aus Diäthyläther / Petroläther 40 - 60°C 2 : 1 zugetropft bis zur beginnenden Trübung. Das Gemisch wird zur Auskristallisation in die Kühltruhe gestellt bei -20°C. Nach dem Absaugen der Kristalle und Trocknung bei 110°C 8 Stunden im Oelpumpenvakuum werden 12,4 g Kristalle erhalten. Aus der Mutterlauge kristallisieren weitere 1,17 g . Die vereinigten Erstkristallisate werden nochmals aus Methanol unter Zugabe von Diäthyläther / Petroläther umkristallisiert. Nach der Trocknung werden erhalten 10,7 g . Schmelzpunkt 179 - 183°C (Zers., ab 150°C Subl. der Base); $[\alpha]_{20}^{D}$ = 25,8°(c = 2 in $CH_3OH$).

Zur Analyse wird nochmals aus Methanol durch Zugabe von Diäthyläther umkristallisiert. Nach Trocknung der Kristalle bei 110°C 8 Stunden im Oelpumpenvakuum werden schwach hygroskopische Kristalle vom Schmp. 187°C (Zers.,ab 158° C Subl. der Base) erhalten; $[\alpha]_{20}^{D}$ = -26°(c = 2 in $CH_3OH$).

Elementaranalyse: ber. für $C_{22}H_{30}N_2O_3$ x HCl; MG = 406,96
C 64,93 %, H 7,68 %, N 6,88 %, Cl 8,71 %, 0,11,79 %
gef.: C 64,60 %, H 7,88 %, N 6,79 %, Cl 8,90 %, 0,11,83 %

Beispiel Nr. 10:

Herstellung von Tetrahydrocorynanthein-Hydrobromid:

0,85 g (50 mmol) 47 % HBr werden mit 20 ml 95 % $C_2H_5OH$ rein verdünnt und 1,85 g (50 mmol) THCN zugegeben. Dabei geht THCN bei Raumtemperatur in Lösung. (Im Kühlschrank fällt das Salz gallertartig aus.) Am Rotationsverdampfer wird bei 50°C zur Trockne eingeengt. Der Rückstand wird in 20 ml $CH_3OH$ p.A. gelöst und unter Eiskühlung wird ein Gemisch Aether / Petroläther 60 - 70°C 2 : 1 bis zur beginnenden Kristallisation zugetropft. Das ausgefallene Salz wird abgesaugt und bei 120°C in der Trockenpistole getrocknet: 1,23 g Kristalle vom Schmp. 240 - 242°C (ab 230°C Zers.); $[\alpha]_{20}^{D}$ = -25,5° (c = 0,2 in $CH_3OH$).
Aus der Mutterlauge werden weitere 0,38 g kristalline Substanz erhalten.
Ausbeute 71,3 % d.Th.

Elementaranalyse: ber. für $C_{22}H_{30}N_2O_3$ x HBr; MG = 451,42
C 58,53 %, H 6,92 %, N 6,20 %, O 10,63 %, Br 17,70 %
gef.: C 58,56 %, H 7,15 %, N 6,07 %, O 10,32 %, Br 17,90 %

Beispiel Nr. 11:

Herstellung von Tetrahydrocorynanthein-Nitrat:

0,48 g (50 mmol) 65 % $HNO_3$ werden mit 20 ml 95 % $C_2H_5OH$ rein verdünnt und 1,85 g (50 mmol) THCN zugegeben. Dabei geht THCN bei Raumtemperatur in Lösung. (Im Kühlschrank erfolgt keine Kristallisation.) Am Rotationsverdampfer wird bei 50°C zur Trockne eingeengt. Der Rückstand wird in 10 ml $CH_3OH$ p.A. gelöst, danach wird unter Eiskühlung Aether bis zur beginnenden Kristallisation zugetropft. Das ausgefallene Salz wird abgesaugt und bei 120°C in der Trockenpistole getrocknet: 1,66 g Kristalle vom Schmp. 182°C (ab 175°C Subl. der Base).

$[\alpha]_D^{20} = -31^0$ (c = 0,2 in $CH_3OH$)
Ausbeute: 76,6 % d.Th.

Elementaranalyse: ber. für $C_{22}H_{30}N_2O_3$ x $HNO_3$; MG = 433,52
C 60,95 %, H 7,20 %, N 9,69 %, O 22,12 %
gef.: C 61,02 %, H 7,43 %, N 9,71 %, O 21,84 %

Beispiel Nr. 12:

Herstellung von Tetrahydrocorynanthein-Hydrogensulfat:

0,49 g (50 mmol) konz. $H_2SO_4$ werden mit 20 ml 95 % $C_2H_5OH$ rein verdünnt, 1,85 g (50 mmol) THCN zugegeben und auf dem Wasserbad bis zum Auflösen erwärmt. Das im Kühlschrank auskristallisierte Salz wird abgesaugt und bei 120° in der Trockenpistole getrocknet: 1,61 g Kristalle vom Schmp. 247 - 248°C; $[\alpha]_D^{20} = -25,5^0$ (c = 0,2 in $CH_3OH$).
Aus der Mutterlauge werden weitere 0,53 g kristalline Substanz erhalten.
Ausbeute: 91,3 % d.Th.

Elementaranalyse ber. für $C_{22}H_{30}N_2O_3$ x $H_2SO_4$; MG = 468,58
C 56,39 %, H 6,88 %, N 5,98 %, O 23,90 %, S 6,84 %
gef.: C 57,00 %, H 7,16 %, N 5,86 %, O 23,38 %, S 6,60 %

Beispiel Nr. 13:

Herstellung von Tetrahydrocorynanthein-Sulfat:

0,245 g (25 mmol) konz. $H_2SO_4$ werden mit 20 ml 95 % $C_2H_5OH$ rein verdünnt und 1,85 g (50 mmol) THCN zugegeben. (Die entstehende milchige Trübung wird beim Kochen mit 200 ml $C_2H_5OH$ am Rückfluß nicht klar.) Am Rotationsverdampfer wird bei 50°C zur Trockne eingeengt. Der Rückstand wird durch Kochen mit 100 ml $CH_3OH$ p.A. am Rückfluß gelöst

und ein Teil des $CH_3OH$ am Rotationsverdampfer abdestilliert. Das im Kühlschrank auskristallisierte Salz wird abgesaugt und bei 120°C in der Trockenpistole getrocknet:
1.66 g Kristalle vom Schmp. 244,5 - 245,5°C;
$[\alpha]_D^{20}$ = -26° (c = 0,2 in $CH_3OH$) .
Aus der Mutterlauge werden weitere 0,25 g kristalline Substanz erhalten.
Ausbeute: 91 % d.Th.

Elementaranalyse ber. für $C_{22}H_{30}N_2O_3$ x 1/2 $H_2SO_4$ ;
MG = 419,54
C 62,99 %, H 7,45 %, N 6,68 %, O 19,07 %, S 3,82 %
gef.: C 62,30 %, H 7,63 %, N 6,35 %, O 19,92 %, S 3,80 %

Beispiel Nr. 14:

Herstellung von Tetrahydrocorynanthein-Methansulfonat:

0,48 g (50 mmol) $CH_3SO_3H$ werden in 20 ml 95 % $C_2H_5OH$ rein gelöst und 1,85 g (50 mmol) THCN zugegeben. Dabei geht THCN bei Raumtemperatur in Lösung. (Im Kühlschrank erfolgt keine Kristallisation.) Am Rotationsverdampfer wird bei 50°C zur Trockne eingeengt. Der Rückstand wird in 20 ml $C_2H_5OH$ gelöst. Das im Kühlschrank auskristallisierte Salz wird abgesaugt und bei 120°C in der Trockenpistole getrocknet; 1,28 g Kristalle vom Schmp. 243 - 250°C ( ab 175°C Subl. der Base); $[\alpha]_D^{20}$ = -27,5° (c = 0,2 in $CH_3OH$). Aus der Mutterlauge werden weitere 0,38 g kristalline Substanz erhalten.
Ausbeute: 71,1 % d.Th.

Elementaranalyse ber. für $C_{22}H_{30}N_2O_3$ x $CH_3SO_3H$; MG=466,62
C 59,20 %, H 7,34 %, N 6,00 %, O 20,57 %, S 6,87 %
gef.: C 59,42 %, H 7,61 %, N 5,82 %, O 20,15 %, S 7,00 %

Beispiel Nr. 15:

Herstellung von Tetrahydrocorynanthein-Dihydrogenphosphat:

0,576 g (50 mmol) 85 % $H_3PO_4$ werden mit 20 ml 95 % $C_2H_5OH$ rein verdünnt und 1,85 g (50 mmol) THCN zugegeben. Dabei geht THCN bei Raumtemperatur in Lösung. (Im Kühlschrank erfolgt keine Kristallisation.) Am Rotationsverdampfer wird bei 50° C zur Trockne eingeengt. Der Rückstand wird in 10 ml 95 % Aethanol rein gelöst, danach wird unter Eiskühlung Aether bis zur beginnenden Kristallisation zugetropft. Das ausgefallene Salz wird abgesaugt und bei 120° C in der Trockenpistole getrocknet: 1,94 g Kristalle vom Schmp. 166 - 167° C; $[\alpha]_D^{20} = -24,5°$ (c = 0,2 in $CH_3OH$) Ausbeute: 76,9 % d.Th.

Elementaranalyse ber. für $C_{22}H_{30}N_2O_3$ x $H_3PO_4$ x 2 $H_2O$;
MG = 504,52

C 52,38 %, H 7,39 %, N 5,55 %, O 28,54 %, P 6,14 %
gef.: C 52,15 %, H 7,08 %, N 5,36 %, O 29,51 %, P 5,90 %

Beispiel Nr. 16:

Herstellung von Tetrahydrocorynanthein-Oxalat:

0,315 g (25 mmol) $C_2H_2O_4$ x 2 $H_2O$ werden in 60 ml $CH_3OH$ p.A. gelöst, 1,85 g (50 mmol) THCN zugegeben und auf dem Wasserbad bis zum Auflösen erwärmt, heiß filtriert. Das im Kühlschrank auskrist. Salz wird abgesaugt und bei 120° C in der Trockenpistole getrocknet: 1,17 g Kristalle vom Schmp. 232 - 33° C (ab 158° C Subl. der Base);
$[\alpha]_D^{20} = -27,81°$ (c = 0,2 in $CH_3OH$)
Aus der Mutterlauge werden weitere 0,36 g kristalline Substanz erhalten. Ausbeute: 72 % d.Th.
Elementaranalyse bez. für $C_{22}H_{30}N_2O_3$ x1/2 $C_2H_2O_4$ x1/2 $H_2O$;
MG = 424,53

C 65,07 %, H 7,60 %, N 6,60 %, 0,20,73 %
gef.: C 65,29 %, H 7,63 %, N 6,58 %, O 20,50 %

## Beispiel Nr. 17:

Herstellung von Tetrahydrocorynantheinsäure:

5 g (13,5 mmol) Tetrahydrocorynanthein werden mit 500 ml 2N-Salzsäure 6 Stunden unter Rückfluß erhitzt. Nach Abkühlung auf Raumtemperatur wird unter externer Kühlung durch portionsweise Zugabe von 2N-Natronlauge der pH auf ca. 9 gebracht. Mit 2 mal 200 ml Methylenchlorid werden 0,14 g nicht hydrolysiertes Tetrahydrocorynanthein extrahiert. Die wässrige Lösung wird mit Eisessig auf pH 6 - 6,5 angesäuert. Nach Zugabe von 20 % Ammoniumsulfat, bezogen auf das Gewicht der Wasserphase, wird diese dreimal mit jeweils 300 ml Methylaethylketon extrahiert. Die vereinigten organischen Phasen werden mit Natriumsulfat getrocknet und am Rotationsverdampfer zur Trockne eingeengt: 4,57 g Rückstand. Dieser wird aus 250 ml Methylaethylketon kristallisiert: 3,83 g Kristalle. Nach Umkristallisation aus 200 ml Methylaethylketon und Trocknung im Vakuum bei 110°C werden erhalten 3,17 g kristalline Tetrahydrocorynantheinsäure vom Schmp. 245 - 248°C (Zers.); $[\alpha]_D^{20}$ =-138° (c = 0,2 in Pyridin). Ausbeute: 64,2 % d.Th.

Elementaranalyse ber. für $C_{21}H_{28}N_2O_3$ x 1/2 $H_2O$;
MG = 365.47

C 69,02 %, H 8,00 %, N 7,66 %, O 15,32 %
gef.: C 68,92 %, H 8,09 %, N 7,43 %, O 15,56 %.

Die freie Säure kann mit anorganischen Basen wie z. B. LiOH, NaOH, KOH, wäßr. Ammoniaklösung oder mit Aminen in ihre entsprechenden Salze übergeführt werden.

Wie schon erwähnt, besitzen die erfindungsgemäßen Verbindungen eine ausgezeichnete blutdrucksenkende Wirkung sowie ausgeprägte vasodilatatorische und das zentrale Nervensystem beeinflussende Eigenschaften; sie werden deshalb vorzugsweise als Antihypertonika verwendet.

Bei den im folgenden beschriebenen pharmakologischen Untersuchungen wurde als Vergleichsverbindung stets Raubasin (= Ajmalicin) herangezogen.

Die blutdrucksenkende Wirkung der erfindungsgemäßen Verbindung THCN und von DHCN wurde an spontan-hypertonen Ratten nach oraler Applikation untersucht und mit derjenigen des Ajmalicin verglichen. Die dabei benutzte Methode für die Messung des systolischen Blutdrucks und der Herzfrequenz an der Ratte ist in Arzneimittel-Forschung 18, 1285 - 1287 (1968) beschrieben. Vor Beginn der Versuche wurden die Tiere an die Meßprozedur gewöhnt.

In einer ersten Versuchsreihe wurde der systolische Blutdruck und die Herzfrequenz zwei Stunden nach oraler Verabreichung der als Hydrochloride vorliegenden Substanzen untersucht. Die Substanzen wurden in 0,2%-Agar suspendiert und in einem Volumen von 10 ml/kg appliziert. Die Ergebnisse sind in den Tabellen 1 bis 3 dargestellt. Daraus geht hervor, daß alle drei Substanzen den Blutdruck dosisabhängig senken.

Die Berechnung der Regressionsgeraden ergibt in allen drei Fällen eine statistisch signifikante Korrelation zwischen Dosis und Ausmaß der Blutdrucksenkung (vergl. Tabelle 4). Für Ajmalicin erreichte die Senkung bei einer Dosis von 56 mg/kg ein Maximum von etwa 18 %, das mit Dosierungen bis 100 mg/kg nicht überschritten werden konnte. Ein Vergleich mit DHCN ergab, daß beide

Dosiswirkungskurven parallel verlaufen (p = 0,30) und daß der Abstand der beiden Geraden mit p = 0,65 nicht signifikant verschieden von 0 ist. Auf der anderen Seite ist die Dosiswirkungskurve für THCN steiler und die Wirkung in höherer Dosierung stärker.

Die Herzfrequenz wird von allen drei Substanzen wenig beeinflußt. Dabei ist weder eine Tendenz noch eine Dosisabhängigkeit zu erkennen.

In einer weiteren Versuchsreihe wurde der zeitliche Verlauf der Blutdrucksenkung untersucht. Dafür wurden spontanhypertone Ratten mit 10 mg/kg DHCN und 5 oder 10 mg/kg THCN per os behandelt und jeweils 6 bzw. 12 Tiere des behandelten Kollektivs zu bestimmten Zeitpunkten nach der Behandlung gemessen.

Die Ergebnisse sind in den Tabellen 5 und 6 dargestellt. DHCN (10 mg/kg) und THCN (5 mg/kg) haben die gleiche Wirkung, deren Maximum ca. 40 Minuten nach der Verabreichung erreicht wird. Danach steigt der Blutdruck wieder auf den Ausgangswert an. THCN (10 mg/kg) hat eine stärkere Wirkung. 20 Min. nach der Verabreichung wird eine maximale Senkung von 30 % erreicht. 6 Stunden nach der Verabreichung ist der Blutdruck immer noch deutlich unter dem Ausgangswert.

Die Herzfrequenz ändert sich nach der Verabreichung von 5 mg/kg THCN nicht. Nach DHCN (10 mg/kg) und THCN (10 mg/kg) ist ein Anstieg zu beobachten, der am Rande der statistischen Signifikanz bleibt.

Die Versuche zeigen, daß insbesondere THCN bei oraler Applikation eine im Vergleich zu Ajmalicin deutlich stärkere und länger anhaltende blutdrucksenkende Wirkung besitzt.

In weiteren Versuchen an verschiedenen Spezies wurde

BAD ORIGINAL

eine erniedrigende Wirkung der erfindungsgemäßen Verbindungen auf den peripheren Widerstand sowie eine vasodilatatorische Wirksamkeit festgestellt. Die erfindungsgemäßen Verbindungen zeigen antagonistische und damit toxizitätshemmende Wirksamkeit gegen Kontraktoren, beispielsweise gegen Noradrenalin und Adrenalin, Nikotin.

In weiteren pharmakologischen Versuchen wurde festgestellt, daß THCN spezifische, das Zentralnervensystem beeinflussende und antiarrhythmische Eigenschaften besitzt. So wurde nach oraler Verabreichung von THCN eine dosisabhängige (bei Dosen zwischen 5 und 20 mg/kg) sedierende und die Körpertemperatur senkende Wirkung an der Maus gefunden. Am gleichen Tier und im selben Dosisbereich zeigt die erfindungsgemäße Verbindung eine Hemmung des durch 5-Hydroxytryptophan verursachten "HeadTwitch"-Syndroms (Versuchsanordnung nach Brit. J. Pharmacol. 20, 106 - 120 (1963)). Daraus folgt, daß THCN eine zentrale anti-serotoninerge Wirkung besitzt.

Weiter wurde gefunden, daß THCN nach oraler Verabreichung an der Maus (in Dosen zwischen 10 und 20 mg/kg) die durch Apomorphin hervorgerufene Stereotypie potenziert (zur Methode vergl. Psychopharmacology 50, 1 (1976)). Dieser Befund deutet auf eine zentrale dopaminerge stimulierende Eigenschaft von THCN hin. DHCN und Ajmalicin zeigen demgegenüber in diesem Dosisbereich keine das Zentralnervensystem beeinflussenden Eigenschaften.

Die antiarrhythmische Wirksamkeit von DHCN und THCN wurde an der Maus und an Ratten geprüft. Hierbei wurde gefunden (vergl. Tabelle 7), daß die Verbindung THCN nach i.p.-Gabe die durch Chloroform verursachten Arrhythmien dosisabhängig (zwischen 10 und 25 mg/kg) hemmt. (Zur Methode vergl. J. Pharmacol. Exptl. Ther. 160, 22 - 31 (1968)). Nach i.v.-Applikation (2-5 mg/kg)

an der Urethan-narkotisierten Ratte schützt ebenfalls THCN gegen Aconitin-induzierte Arrhythmien (zur Methode vergl. Basic. Res. Cardiol. 66, 73 - 79 (1973)). Ajmalicin zeigt an beiden Modellen dagegen keine antiarrhythmische Wirksamkeit.

Die Toxizität der erfindungsgemäßen Verbindungen ergibt sich aus den in Tabelle 8 angegebenen $LD_{50}$-Werten für intravenöse, intraperitoneale und orale Applikation. Daraus ergibt sich, daß die Toxizitäten von THCN, DHCN und Ajmalicin nahezu gleich sind.

Aus den vorstehend beschriebenen pharmakologischen und toxikologischen Untersuchungen ergibt sich jedoch, daß die Wirkungsdauer und -stärke der erfindungsgemäßen Verbindungen und des DHCN größer ist als die der bekannten Vergleichsverbindung Ajmalicin. THCN besitzt zusätzlich eine günstige, spezifisch das Zentralnervensystem beeinflussende und antiarrhythmische Wirkung. Aufgrund des aufgezeigten pharmakologischen Wirkungsprofils sind die erfindungsgemäßen Verbindungen zur Therapie folgender Krankheiten geeignet:

1. zentrale und/oder periphere Durchblutungsstörungen
2. Arrhythmie
3. Krankheiten des Zentralnervensystems, deren Symptome auf eine Störung des Serotonin- und/oder Dopaminstoffwechsels zurückzuführen sind,
4. Hypertonie
5. Kombinationen der vorstehend genannten Krankheiten.

TABELLE 1

Ajmalicin

| Dosis | n | Gewicht | systolischer Blutdruck mm Hg | | | | Herzfrequenz Schlag/min | | | |
|---|---|---|---|---|---|---|---|---|---|---|
| | | | Vorwert | Nachwert | Δ | Δ % | Vorwert | Nachwert | Δ | Δ % |
| 18 mg/kg | 3 | 211 ± 3 | 210 ± 6 | 198 ± 7 | -12 ± 2 | -5,6 ± 1 | 462 ± 42 | 492 ± 27 | 30 ± 15 | 7,3 ± 4,3 |
| 40 mg/kg | 6 | 202 ±3,8 | 198 ± 6 | 173 ± 7 | -25 ± 6 | -12,4 ± 3 | 462 ± 17 | 513 ± 11 | 51 ± 19 | 11,9 ± 4,4 |
| 56 mg/kg | 5 | 213 ± 6 | 209 ± 8 | 172 ± 4 | -37 ± 5 | -17,5 ± 2,1 | 518 ± 6 | 522 ± 9 | 4 ± 9 | 0,8 ± 1,6 |
| 86 mg/kg | 5 | 218 ± 2 | 201 ± 6 | 165 ±11 | -36 ±12 | -17,7 ± 6,1 | 497 ± 9 | 511 ± 4 | 14 ± 2 | 2,9 ± 2,0 |
| 100 mg/kg | 5 | 218 ± 4 | 198 ± 6 | 165 ± 5 | -33 ± 9 | -16,3 ± 4 | 489 ± 5 | 515 ± 9 | 26 ± 11 | 5,3 ± 2,0 |

Tabelle 1: Systolischer Blutdruck und Herzfrequenz der spontanhypertonen Ratte vor und 2 Stunden nach Verabreichung von verschiedenen Dosen von Ajmalicin·HCl (in 0,2% Agar suspendiert, 10 ml/kg p.o.)

Mittelwerte ± Standard Fehler

n = Zahl der Tiere

0086416

TABELLE 2

Dihydrocorynanthein

| Dosis | n | Gewicht | systolischer Blutdruck mm Hg | | | | Herzfrequenz Schlag/min | | | |
|---|---|---|---|---|---|---|---|---|---|---|
| | | | Vorwert | Nachwert | Δ | Δ % | Vorwert | Nachwert | Δ | Δ % |
| 4 mg/kg | 5 | 189 ± 3 | 199 ± 5 | 187 ± 6 | -12,7 ± 7 | -5,8 ± 3,3 | 479 ± 9 | 485 ± 14 | 6 ± 20 | 1,5 ± 4,2 |
| 8,6 mg/kg | 6 | 203 ± 5 | 200 ± 6 | 187 ± 5 | -13 ± 9 | -6,2 ± 4,0 | 472 ± 17 | 442 ± 19 | -30± 18 | -6,2 ± 3,7 |
| 18 mg/kg | 12 | 206 ± 3 | 202 ± 4 | 181 ± 3 | -21 ± 5 | -10,1± 2,1 | 496 ± 10 | 492 ± 12 | -4 ± 14 | -0,6 ± 2,9 |
| 32 mg/kg | 5 | 202 ± 4 | 204 ± 2 | 175 ± 10 | -29 ± 8 | -14,6± 4,1 | 493 ± 10 | 477 ± 5 | -16± 11 | -3,1 ± 2,2 |
| 56 mg/kg | 6 | 205 ± 4 | 193 ± 3 | 152 ± 6 | -41 ± 5 | -21,4± 2,7 | 491 ± 22 | 502 ± 14 | 3 ± 21 | 2,9 ± 3,8 |

Tabelle 2: Systolischer Blutdruck und Herzfrequenz der spontanhypertonen Ratte vor und zwei Stunden nach Verabreichung von verschiedenen Dosen von Dihydrocorynanthein (in 0,2% Agar suspendiert, 10 ml/kg p.o.)

Mittelwerte ± Standard Fehler

n = Zahl der Tiere

TABELLE 3

Tetrahydrocorynanthein

| Dosis | n | Gewicht | systolischer Blutdruck mm Hg | | | | Herzfrequenz Schlag/min | | | |
|---|---|---|---|---|---|---|---|---|---|---|
| | | | Vorwert | Nachwert | Δ | Δ % | Vorwert | Nachwert | Δ | Δ % |
| 5 mg/kg | 12 | 205 ± 2 | 193 ± 6 | 178 ± 4 | -15 ± 3 | -7,7 ± 1,8 | 502 ± 12 | 508 ± 7 | 6 ± 12 | 1,2 ± 7,1 |
| 10 mg/kg | 12 | 213 ± 5 | 196 ± 3 | 168 ± 5 | -28 ± 6 | -14,3± 4,7 | 498 ± 6 | 519 ± 9 | 21 ± 11 | 4,2 ± 3,3 |
| 18 mg/kg | 6 | 208 ± 4 | 198 ± 7 | 165 ± 13 | -33 ± 11 | -16,5± 5,8 | 482 ± 17 | 495 ± 6 | 13 ± 12 | 3,3 ± 2,8 |
| 30 mg/kg | 6 | 204 ± 3 | 189 ± 8 | 146 ± 5 | -43 ± 5 | -21,9± 3,9 | 471 ± 20 | 493 ± 10 | 22 ± 27 | 4,7 ± 6,6 |
| 40 mg/kg | 6 | 203 ± 2 | 206 ± 4 | 132 ± 9 | -74 ± 10 | -35,7± 4,7 | 473 ± 22 | 472 ± 14 | -1 ± 29 | 1,0 ± 6,6 |

Tabelle 3: Systolischer Blutdruck und Herzfrequenz der spontanhypertonen Ratte vor und zwei Stunden nach Verabreichung von verschiedenen Dosen von Tetrahydrocorynanthein (in 0,2% Agar suspendiert, 10 ml/kg p.o.)
Mittelwerte ± Standard Fehler
n = Zahl der Tiere

TABELLE 4

| Substanz | Korrelation r | p | Parameter der Regressionsgerade | |
|---|---|---|---|---|
| | | | Neigung | Interzept |
| DHCN | 0,587 | $<$0,001 | 0,309 $\pm$ 0,075 | 4,31 $\pm$ 2,18 |
| THCN | 0,655 | $<$0,001 | 0,608 $\pm$ 0,115 | - 7,99 $\pm$ 2,50 |
| Ajmalicin * | 0,660 | $<$0,01 | 0,312 $\pm$ 0,103 | - 0,05 $\pm$ 4,45 |

Tabelle 4: Regressionsanalyse der Dosis-Wirkungsbeziehungen für Ajmalicin, Dihydrocorynanthein und Tetrahydrocorynanthein.
Werte $\pm$ Standard Fehler

*) Nur für die Dosierungen 18 bis 56 mg/kg

TABELLE 5

| Substanz (Dosis) | Ausgangswert (100%) mm Hg | Prozentuale Abnahme zum Zeitpunkt t = | | | | | | |
|---|---|---|---|---|---|---|---|---|
| | | 10 min | 20 min | 40 min | 80 min | 2 h | 4 h | 6 h |
| DHCN 10 mg/kg | 180,6 ± 9,4 | 10,6*** ± 4,1 | 14,5*** ± 3,2 | 14,5*** ± 1,9 | 6,1** ± 2,8 | 3,7* ± 3,6 | 0,8 ± 4,2 | 1,4 ± 1,3 |
| THCN 5 mg/kg | 193,4 ± 7,3 | 11,1*** ± 2,0 | 13,8*** ± 2,0 | 12,8*** ± 3,1 | 13,8*** ± 2,2 | 8,1*** ± 2,2 | - 0,2 ± 1,8 | 4,0* ± 2,0 |
| THCN 10 mg/kg | 196,7 ± 3,2 | 13,5*** ± 1,2 | 29,3*** ± 2,3 | 27,8*** ± 2,6 | 23,0*** ± 2,1 | 14,4*** ± 2,5 | 14,0*** ± 2,8 | 15,9*** ± 2,6 |
| Ajmalicin 20 mg/kg | 188,6 ± 9,0 | 9,5*** ± 3,8 | 13,0*** ± 2,4 | 14,2*** ± 4,6 | 9,8*** ± 3,3 | 5,8** ± 2,4 | - 1,2 ± 4,8 | - 0,5 ± 1,4 |

Tabelle 5: Zeitlicher Verlauf der Blutdrucksenkung nach Verabreichung von Dihydrocorynanthein und Tetrahydrocorynanthein an der spontanhypertonen Ratte.

Mittelwerte ± Standard Fehler — *p < 0,05

Zahl der Tiere siehe Tabelle 6 — **p < 0,01

***p < 0,001

TABELLE 6

| Substanz (Dosis) | n | Ausgangs-wert | Herzfrequenz zum Zeitpunkt t: | | | | | | |
|---|---|---|---|---|---|---|---|---|---|
| | | | 10 min | 20 min | 40 min | 80 min | 2 h | 4 h | 6 h |
| DHCN (10 mg/kg) | 6 | 479 ± 27 | 504 ± 15 | 515 ± 9 | 500 ± 8 | 516 ± 6 | 521 ± 3 | 517 ± 8 | 507 ± 9 |
| THCN ( 5 mg/kg) | 12 | 502 ± 12 | 504 ± 12 | 492 ± 7 | 499 ± 16 | 503 ± 10 | 508 ± 7 | 502 ± 9 | 489 ± 8 |
| THCN (10 mg/kg) | 12 | 499 ± 16 | 519* ± 7 | 518 ± 9 | 499 ± 12 | 520* ± 7 | 519* ± 6 | 519 ± 8 | 503 ± 8 |
| Ajmalicin (20 mg/kg) | 6 | 492 ± 15 | 503 ± 12 | 505 ± 9 | 482 ± 9 | 481 ± 10 | 472 ± 10 | 499 ± 7 | 491 ± 17 |

Tabelle 6: Zeitlicher Verlauf der Herzfrequenz von spontanhypertonen Ratten nach Verabreichung von Dihydrocorynanthein und Tetrahydrocorynanthein.

Mittelwerte ± Standard Fehler    $^{*}p \leqslant 0{,}05$

n = Zahl der Tiere

## TABELLE 7

### Antiarrhythmische Wirkung bei der Maus

| Substanz | Dosis (mg/kg, i.p.) | n | Hemmung in % der $CHCl_3$ Arrhythmie |
|---|---|---|---|
| Ajmalicin | 25 | 10 | 0 |
| DHCN | 25 | 10 | 80 |
| | 15 | 10 | 10 |
| | 10 | 10 | 0 |
| THCN | 25 | 10 | 90 |
| | 15 | 10 | 60 |
| | 10 | 10 | 10 |

(n = Anzahl der untersuchten Tiere)

Tabelle 8

| Test-Substanz | Verabreichung | $LD_{50}$ (mg/kg) | Zahl der Tiere |
|---|---|---|---|
| THCN | i.v. | 14,1 (12,6 - 15,6) | 60 |
| | p.o. | 283,0 (267,0 - 300,0) | 50 |
| | i.p. | 133,3 (118,4 - 169,8) | 50 |
| DHCN | i.v. | 13,0 (11,2 - 15,0) | 50 |
| | p.o. | 210,0 (170,7 - 258,3) | 50 |
| | i.p. | 98,0 ( 83,0 - 115,7) | 50 |
| Ajmalicin | i.v. | 14,0 ( 12,6 - 15,5 ) | 50 |
| | p.o. | 360,0 (315,5 - 410,4) | 50 |
| | i.p. | 90,0 ( 74,4 - 108,9) | 50 |

$LD_{50}$-Werte (nach Litchfield u. Wilcoxon) an der Maus

P 2596 EP 4.2.1983

## Patentansprüche

1. Corynanthein-Derivate der allgemeinen Formel I,

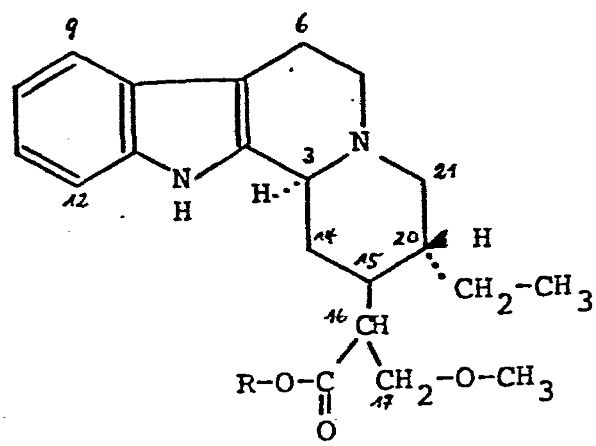

(I)

worin R ein Wasserstoff- oder Alkaliatom, $NH_4$- oder den Rest eines Amins oder eine Alkylgruppe mit 1 bis 4 C-Atomen bedeutet, sowie deren physiologisch unbedenkliche Säureadditionssalze.

2. Tetrahydrocorynanthein der Formel I, worin R eine Methylgruppe ist, sowie dessen physiologisch unbedenkliche Säureadditionssalze.

3. Tetrahydrocorynantheinsäure sowie deren Salze mit anorganischen Basen und mit physiologisch unbedenklichen Aminen.

4. Verfahren zur Herstellung der Corynanthein-Derivate gemäß Anspruch 1 oder 2, dadurch gekennzeichnet, daß

a) Corynanthein und/oder Dihydrocorynanthein oder diese beiden Substanzen enthaltende Naturstoffgemische katalytisch hydriert werden,
b) das so entstandene Tetrahydrocorynanthein zur freien Säure (R = H) verseift oder aber umgeestert wird (R = $C_2H_5-$, $C_3H_7-$, $C_4H_9-$),
c) die freie Säure (R = H) ggfs. in ihre ($C_1$ bis $C_4$)-Alkylester oder in ihre Salze mit anorganischen Basen und mit physiologisch unbedenklichen Aminen übergeführt wird
d) und ggfs. die nach Durchführung der vorstehend genannten Verfahrensschritte (a), (b) und/oder (c) erhaltenen Reaktionslösungen mit einer anorganischen Säure oder einer starken organischen Säure zur Bildung der entsprechenden Säureadditionssalze versetzt wird.

5. Verfahren nach Anspruch 4, dadurch gekennzeichnet, daß die Hydrierung unter Verwendung von Edelmetallkatalysatoren, insbesondere von Pd-, Pt- oder Rh-Katalysatoren, oder von aktiven Nickelkatalysatoren durchgeführt wird.

6. Verfahren nach Anspruch 5, dadurch gekennzeichnet, daß als Katalysator Pd auf Kohle, Ni auf Kieselgur oder Raney-Nickel verwendet wird.

7. Verfahren nach einem der Ansprüche 4, 5 oder 6, dadurch gekennzeichnet, daß die Hydrierung in alkoholischer oder wäßrig-alkoholischer Lösung der als Ausgangsmaterial verwendeten Alkaloide durchgeführt wird.

8. Verfahren nach einem der Ansprüche 4 bis 7, dadurch gekennzeichnet, daß die Hydrierung bei einem Druck von 50 bis 110 bar und einer Temperatur von 20 bis 100°C durchgeführt wird.

9. Pharmazeutische Zubereitung, enthaltend eine Verbindung gemäß einem der Ansprüche 1 bis 3 sowie übliche Träger und Zusatzstoffe.

10. Verwendung von Dihydrocorynanthein als Antihypertonikum.